# EUROPÄISCHE PATENTSCHRIFT

(11) **EP 0 364 612 B1**
(45) Veröffentlichungstag und Bekanntmachung des Hinweises auf die Patenterteilung: **10.02.1993**
(21) Anmeldenummer: 88117261.3
(22) Anmeldetag: 17.10.1988
(51) Int. Cl.: A61B 6/03

(54) **Computertomograph**
Computerised tomography apparatus
Appareil de tomographie assisté par ordinateur

(43) Veröffentlichungstag der Anmeldung: 25.04.1990
(73) Patentinhaber: SIEMENS AKTIENGESELLSCHAFT, 80333 München (DE)
(72) Erfinder: Hahn, Günter, Dipl.-Ing.(FH), D-8524 Neunkirchen am Brand (DE)

(56) Entgegenhaltungen:
- EP-A- 0 077 471
- FR-A- 2 296 893

## Beschreibung

Die Erfindung betrifft einen Computertomographen mit einem Strahlendetektor aus einer Reihe von Detektorelementen und einem Datenerfassungssystem für die Detektorsignale, das einen Multiplexer für das Abfragen der Signale der Detektorelemente aufweist.

Es sind Computertomographen dieser Art bekannt, die einen Drehrahmen aufweisen, der eine Meßöffnung für den Patienten umschließt und auf dem ein Röntgenstrahler und der Strahlendetektor angeordnet sind (DE-A-30 19 606). Der Röntgenstrahler sendet ein fächerförmiges Röntgenstrahlenbündel aus, das eine Transversalschicht des Patienten durchsetzt und auf dem Strahlendetektor auftrifft. Zur Durchstrahlung des Patienten unter verschiedenen Richtungen wird der Drehrahmen um 360° um den Patienten gedreht und es werden die Detektorsignale bei bestimmten Winkelstellungen, z.B. bei jedem Winkelgrad, durch den Multiplexer aufeinanderfolgend abgefragt. Die Abfrage erfolgt dabei in einer festen Reihenfolge. Aufgrund dieser starren Abfrage können schon kleine Fehler, verursacht durch den Auslesevorgang für die einzelnen Detektorkanäle, in dem von einem Computer aus den Detektorsignalen erzeugten Bild sichtbar werden.

Der Erfindung liegt die Aufgabe zugrunde, einen Computertomographen der eingangs genannten Art so auszubilden, daß die störende Eigenschaft, daß schon kleinste Fehler, verursacht durch den Auslesevorgang für die einzelnen Detektorkanäle, zu Bildfehlern führen, beseitigt ist.

Diese Aufgabe ist erfindungsgemäß dadurch gelöst, daß der Multiplexer so gesteuert ist, daß die Abfrage in einer pseudo-zufälligen Reihenfolge erfolgt. Diese pseudo-zufällige Abfrage-Reihenfolge, die an die Stelle einer festen Abfrage-Reihenfolge tritt, stellt sicher, daß die geschilderten Bildfehler vermieden werden.

Eine Weiterbildung der Erfindung besteht darin, daß bei den dem Multiplexer nachgeschalteten Analog-Digital-Wandlern die Zuordnung der einzelnen Kanäle der Detektorelemente zu den Analog- Digital-Wandlern wechselt. Dadurch werden auch Bildfehler aufgrund von Analog-Digital-Wandlern mit unterschiedlichem Übertragungsverhalten weitgehend vermieden.

Die Erfindung ist nachfolgend anhand eines in der Zeichnung dargestellten Ausführungsbeispieles näher erläutert. Es zeigen:
- Fig. 1: einen Computertomographen zur Erläuterung des Erfindungsgedankens, und
- Fig. 2: eine Einzelheit des Computertomographen gemäß Fig. 1.

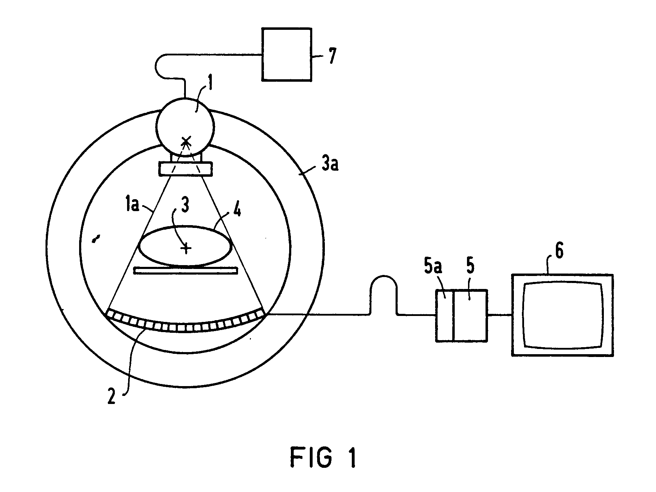

In der Fig. 1 ist ein Computertomograph mit einer Röntgenröhre 1 als Strahlenquelle und einem Strahlendetektor 2, der großenordnungsmäßig über 100, z.B. 512 einzelne Detektoren in einer Reihe angeordnet aufweist, dargestellt. Die Röntgenröhre 1 sendet ein fächerförmiges Strahlenbündel 1a aus, dessen Querschnittsausdehnung senkrecht zur untersuchten Schichtebene gleich der Schichtstärke und in der Schichtebene so groß ist, daß das ganze Aufnahmeobjekt von Strahlung durchsetzt wird. Der Strahlendetektor 2 ist um den Fokus der Röntgenröhre 1 gekrümmt. Die Meßanordnung 1, 2 ist um eine Achse 3, die etwa mit der Längsachse des Aufnahmeobjektes 4 zusammenfällt, mit Hilfe eines Drehrahmens 3a drehbar. Die Anzahl der Detektoren des Strahlendetektors 2 ist der gewünschten Bildauflösung entsprechend gewählt, so daß aufgrund einer Drehung der Meßanordnung 1, 2 von einem Computer 5 die Schwächungswerte einer Bildpunktmatrix der durchstrahlten Transversalschicht des Aufnahmeobjektes 4 berechnet und als Bild auf einem Sichtgerät 6 wiedergegeben werden können. Die Röntgenröhre 1 wird von einem Röntgengenerator 7 gespeist.

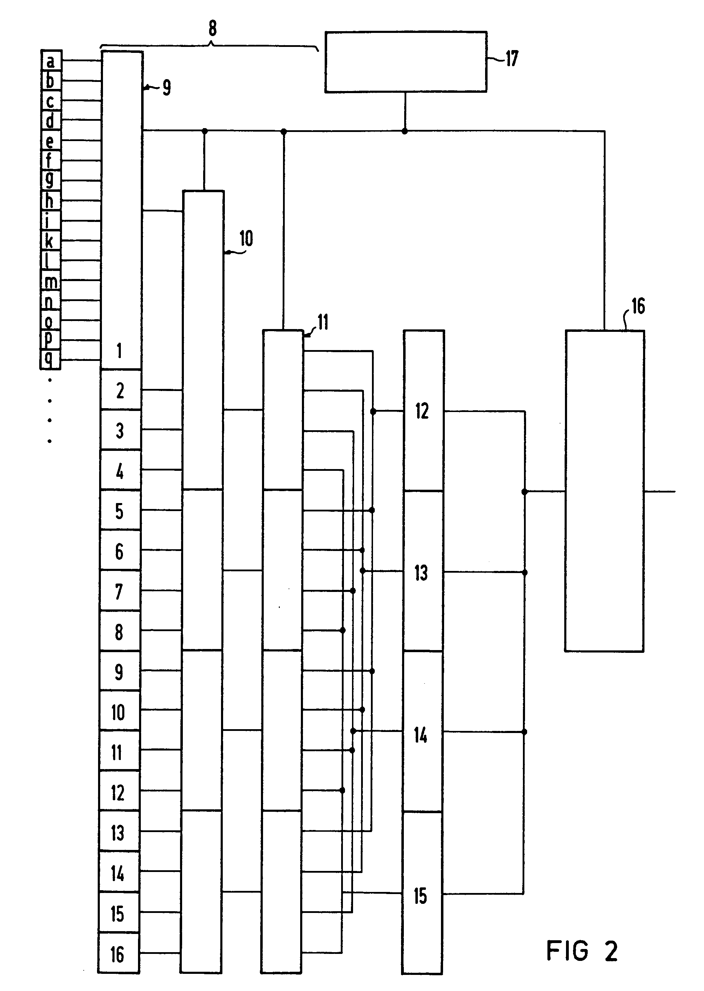

Jedem Detektorelement des Strahlendetektors 2 ist ein Meßkanal zugeordnet, der zum Computer 5 führt. In diesem Meßkanal sind Verstärkerschaltungen, Multiplexer und Analog-Digital-Wandler vorgesehen, die ein Datenerfassungssystem 5a bilden. Das Datenerfassungssystem 5a ist anhand der Fig. 2 näher erläutert.

In der Fig. 2 ist eine Reihe von Detektorkanälen a bis q usw. dargestellt, die durch eine erste Multiplexerebene 9 eines Multiplexers 8 in Gruppen von 16 Kanälen 1 bis 16 zusammengefaßt werden. Die zweite Multiplexerebene 10 faßt die 16-Gruppen der Multiplexerebene 9 zu vier Analog-Bussen zusammen. Die dritte Multiplexerebene 11 verteilt die vier Analog-Busse in einer frei wählbaren Reihenfolge auf vier Analog-Digital-Wandler 12, 13, 14 und 15. Die Adressierung der Multiplexerebenen 9, 10, 11 und eines den Analog-Digital-Wandlern 12 bis 15 nachgeschalteten Speichers 16 erfolgt durch eine Steuervorrichtung 17.

Die Steuervorrichtung 17 ist so programmiert, daß für die Adressen der Multiplexerebenen 9 und 10 ein Wechsel der Abfragereihenfolge nach einem vollständigen Auslesevorgang erfolgt. Für die Adresse der Multiplexerebene 11 erfolgt ein Wechsel der Buszuordnung nach jedem Wandlerzyklus.

Für einen Bildaufbau sind mehrere (ca. 400 bis 2000) Abfragezyklen erforderlich. Durch den beschriebenen Aufbau ist es möglich, die Abfragereihenfolge für die Kanäle a usw. sowie die Zuordnung der einzelnen Kanäle a usw. zu den Analog-Digital-Wandlern 12 bis 15 pseudo-zufällig zu erzeugen.

In dem nachfolgenden Speicher 16 werden die erzeugten digitalen Werte in der Reihenfolge 1, 2, 3 ... n-1, n abgespeichert. Somit steht für die Weiterverarbeitung durch den Computer 5 und die Bilderzeugung ein sortierter Wertesatz zur Verfügung.

## Patentansprüche

1. Computertomograph mit einem Strahlendetektor (2) aus einer Reihe von Detektorelementen und einem Datenerfassungssystem (5a) für die Detektorsignale, das einen Multiplexer (8) für die Abfrage der Signale der Detektorelemente aufweist, **dadurch gekennzeichnet,** daß der Multiplexer (8) so gesteuert ist, daß die Abfrage in einer pseudo-zufälligen Reihenfolge erfolgt.

2. Computertomograph nach Anspruch 1, **dadurch gekennzeichnet,** daß bei den dem Multiplexer (8) nachgeschalteten Analog-Digital-Wandlern (12 bis 15) die Zuordnung der einzelnen Kanäle der Detektorelemente zu den Analog-Digital-Wandlern (12 bis 15) wechselt.

3. Computertomograph nach Anspruch 2, **dadurch gekennzeichnet,** daß die Analog-Digital-Wandler (12 bis 15) an einem Speicher (16) angeschlossen sind, in dem die erzeugten digitalen Werte in der Reihenfolge (1, 2, 3 ... n-1, n) abgespeichert werden.

## Claims

1. Computer tomography apparatus having a radiation detector (2) consisting of a series of detector elements and a data acquisition system (5a) for the detector signals, which has a multiplexer (8) for the interrogation of the signals of the detector elements, characterised in that the multiplexer (8) is controlled in such a way that the interrogation occurs in a pseudo-random sequence.

2. Computer tomography apparatus according to claim 1, characterised in that in the case of the analog-to-digital converters (12 to 15) which are subsequently connected to the multiplexer (8) the coordination of the individual channels of the detector elements with the analog-to-digital converters (12 to 15) changes.

3. Computer tomography apparatus according to claim 2, characterised in that the analog-to-digital converters (12 to 15) are connected to a memory (16) in which the digital values produced are stored in the sequence (1, 2, 3 ... n-1, n).

## Revendications

1. Appareil de tomographie assisté par ordinateur comportant un détecteur de rayonnement (2) constitué par une série d'éléments de détection et un système d'acquisition de données (5a) pour les signaux du détecteur, qui comporte un multiplexeur (8) pour l'interrogation des signaux des éléments de détection, caractérisé par le fait que le multiplexeur (8) est commandé de telle sorte que l'interrogation s'effectue selon une séquence pseudo-aléatoire.

2. Appareil de tomographie assisté par ordinateur suivant la revendication 1, caractérisé par le fait que, dans le cas des convertisseurs analogique/numérique (12 à 15) branchés en aval du multiplexeur (8), l'association des différents canaux des éléments de détection aux convertisseurs analogique/numérique (12 à 15) est alternée.

3. Appareil de tomographie assisté par ordinateur suivant la revendication 2, caractérisé par le fait que les convertisseurs analogique/numérique (12 à 15) sont raccordés à une mémoire (16), dans laquelle les valeurs numériques produites sont mémorisées selon la séquence (1, 2, 3, ..., n-1, n).
